(19)

Europäisches
Patentamt
European
Patent Office
Office européen
des brevets

(11) **EP 3 536 224 A1**

(12) **EUROPEAN PATENT APPLICATION**

(43) Date of publication:
**11.09.2019 Bulletin 2019/37**

(51) Int Cl.:
***A61B 5/00*** (2006.01)

(21) Application number: **18160467.9**

(22) Date of filing: **07.03.2018**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AE Eindhoven (NL)**

(72) Inventors:
• **Ras, Arno
5656 AE Eindhoven (NL)**

• **Hermans, Walter
5656 AE Eindhoven (NL)**
• **Westerhof, Wilko
5656 AE Eindhoven (NL)**
• **Varghese, Babu
5656 AE Eindhoven (NL)**

(74) Representative: **de Haan, Poul Erik et al
Philips International B.V.
Philips Intellectual Property & Standards
High Tech Campus 5
5656 AE Eindhoven (NL)**

(54) **SYSTEM FOR MEASURING SKIN PORES**

(57)      There is provided a system (10) for measuring skin pores. The system (10) comprises a device (100). The device (100) comprises a light source (104) configured to emit light (106) to illuminate skin (102) with an angle of incidence in a range from 40 to 80 degrees. The device (100) comprises an imaging sensor (108) configured to detect light (110) reflected from the skin (102) and convert the detected light (110) into an image of the skin (102). The system comprises a processor (111) configured to acquire the image of the skin (102) from the imaging sensor (108) and process the acquired image to measure skin pores.

Fig. 1

**Description**

FIELD OF THE INVENTION

The disclosure relates to a system for measuring skin pores and a method for measuring skin pores.

BACKGROUND OF THE INVENTION

In the skincare field, it is valuable to acquire images of the skin. For example, images of the skin can be useful for monitoring the state of the skin (such as any deterioration or improvement in the state of the skin) and to detect any skin conditions. Some existing systems for imaging skin comprise a light source for illuminating the skin while an image is acquired by an imaging sensor of the system. For example, US 8,346,347 discloses a system and method in which one or more skin characteristics are determined by analyzing radiation backscattered from a skin region illuminated.

However, while there exist such systems for imaging skin, the existing systems can suffer from poor image quality. For example, the images acquired by the existing systems are often not of a high enough resolution for features of the skin (such as skin pores, skin texture, fine lines and wrinkles, skin spots, impurities, irregularities, abnormalities, etc.) to be resolved to an adequate degree.

There is thus a need for an improved system and method for imaging skin, which aims to address the existing problems.

SUMMARY OF THE INVENTION

As noted above, a limitation with existing systems is that the images acquired by such systems are often not of a high enough resolution for features of the skin (such as skin pores, skin texture, fine lines and wrinkles, skin spots, impurities, irregularities, abnormalities, etc.) to be adequately resolved. It would thus be valuable to have an improved system and method for imaging skin, which aims to address the existing problems.

Therefore, according to a first aspect, there is provided a system for measuring skin pores. The system comprises a device. The device comprises a light source configured to emit light to illuminate skin with an angle of incidence in a range from 40 to 80 degrees. The device also comprises an imaging sensor configured to detect light reflected from the skin and convert the detected light into an image of the skin. The system further comprises a processor configured to acquire the image of the skin from the imaging sensor and process the acquired image to measure skin pores.

In some embodiments, the light source may be configured to emit light to illuminate the skin with an angle of incidence in a range from 50 to 70 degrees.

In some embodiments, the light source may have a radiation angle in a range from 5 to 40 degrees. In some embodiments, the light source may have a radiation angle in a range from 10 to 30 degrees. In these embodiments, the radiation angle may be the angle at which an intensity of the light emitted by the light source is half that of a maximum intensity of the light emitted by the light source.

In some embodiments, the light source may be configured to emit light to uniformly illuminate the skin.

In some embodiments, the imaging sensor may be further configured to calibrate the image of the skin using flat field correction.

In some embodiments, the light source may comprise a plurality of light emitters. In some embodiments, the plurality of light emitters may be positioned in a circular arrangement. In some embodiments, the plurality of light emitters may be configured to sequentially emit light to illuminate the skin.

In some embodiments, the device may further comprise an optical component positioned such that the light emitted from the light source passes through the optical component before illuminating the skin, wherein the optical component may be configured to collimate the light emitted from the light source.

In some embodiments, the optical component may comprise any one or more of: a light guide, an off-axis lens, a periscope, a white cap with diffuse internal reflection, and a Fresnel lens.

In some embodiments, the device may further comprise a housing configured to house the light source and the imaging sensor. In some of these embodiments, the housing may comprise an aperture through which the light source may be configured to emit light and the imaging sensor may be configured to detect light reflected from the skin. In some embodiments, the aperture may have a stop size in a range from 0.2 millimeters to 0.6 millimeters.

In some embodiments, a distance between the light source and the imaging sensor may be greater than 12 millimeters.

According to a second aspect, there is provided a method for measuring skin pores. The method comprises emitting light to illuminate skin with an angle of incidence in a range from 40 to 80 degrees, detecting light reflected from the skin, converting the detected light into an image of the skin, and processing the image of the skin to measure skin pores.

According to a third aspect, there is provided a computer program product comprising a computer readable medium, the computer readable medium having computer readable code embodied therein, the computer readable code being configured such that, on execution by a suitable computer or processor, the computer or processor is caused to perform the method described above.

According to the aspects and embodiments described above, the limitations of existing systems are addressed. In particular, according to the above-described aspects and embodiments, images of improved resolution (and thus improved quality) can be acquired. The resolution of the images that is achieved is optimum for skin pores to be adequately resolved in the images. Thus, the images can be used to provide more accurate skin monitoring and to more reliably detect skin conditions. There is thus provided an improved system and method for imaging skin, which overcomes existing problems.

These and other aspects will be apparent from and elucidated with reference to the embodiment(s) described hereinafter.

BRIEF DESCRIPTION OF THE DRAWINGS

Exemplary embodiments will now be described, by way of example only, with reference to the following drawings, in which:

Fig. 1 is a block diagram of a system according to an embodiment;
Figs. 2A-G are example images acquired by an imaging sensor of a device according to an embodiment;
Fig. 3 illustrates example images acquired by an imaging sensor of a device according to another embodiment;
Fig. 4 is a graphical illustration of pore contrast measured for different angles of incidence according to an embodiment;
Fig. 5 illustrates a radiation angle range for a light source of a device according to an embodiment;
Fig. 6 illustrates example images acquired by an imaging sensor of a device according to another embodiment;
Fig. 7 is a block diagram of an example device according to an embodiment;
Fig. 8 is a flow chart illustrating a method according to an embodiment.

DETAILED DESCRIPTION OF EMBODIMENTS

As noted above, there is provided an improved system for imaging skin, which overcomes existing problems. More specifically, there is provided a system for measuring skin pores, e.g. for measuring the size of skin pores.

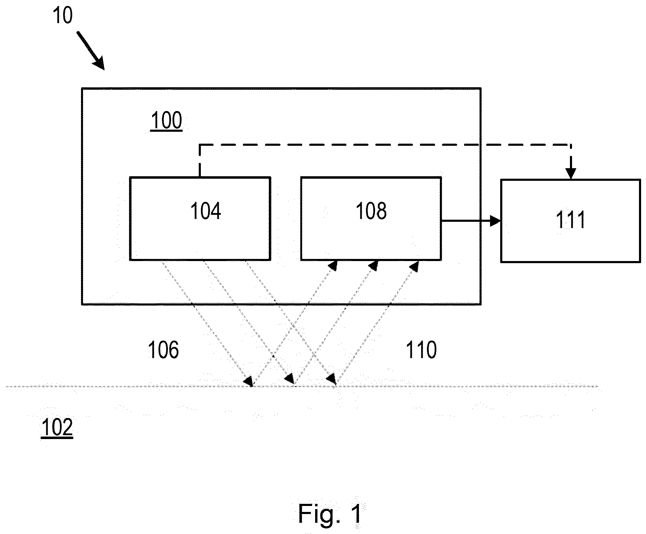

Fig. 1 illustrates a system 10 for measuring skin pores according to an embodiment. As illustrated in Fig. 1, the system 10 comprises a device 100. The device 100 is suitable for imaging skin 102. As also illustrated in Fig. 1, the device 100 comprises a light source 104. The light source 104 is configured to emit light 106 to illuminate skin 102. In particular, the light source 104 is configured to emit light 106 to illuminate skin 102 with an angle of incidence (AOI) in a range from 40 to 80 degrees. The angle of incidence referred to herein is the angle that the light 106 emitted from the light source 104 (that is, the light that is incident on the skin 102 or that illuminates the skin 102) makes with the normal at a point of incidence on the skin 102.

In some embodiments, the light source 104 can, for example, comprise a semiconductor light source such as a light emitting diode (LED), a resonant cavity light emitting diode (RCLED), a vertical cavity laser diode (VCSELs), an edge emitting laser, or any other semiconductor light source. Alternatively or in addition, in some embodiments, the light source 104 can, for example, comprise an organic light source such as a passive-matrix (PMOLED), an active-matrix (AMOLED), or any other organic light source. Alternatively or in addition, in some embodiments, the light source 104 can, for example, comprise a solid state light source. However, although examples for the type of light source 104 have been provided, it will be understood that other types of light source may be used.

As illustrated in Fig. 1, the device 100 further comprises an imaging sensor 108. The imaging sensor 108 is configured to detect light 110 reflected from the skin 102 and convert the detected light 110 into an image of the skin 102. In some embodiments, the imaging sensor 108 described herein may be, for example, a camera sensor. In some embodiments, the imaging sensor 108 may comprise a focusing lens. Alternatively or in addition, in some embodiments, the imaging sensor 108 may comprise an aperture. In some embodiments, the aperture of the imaging sensor 108 may have a stop size in a range from 0.2 mm to 0.6 mm. Although examples have been provided for the imaging sensor 108, it will be understood that any other type of imaging sensor is also possible.

As illustrated in Fig. 1, the system 10 further comprises a processor 111. As illustrated in Fig. 1, in some embodiments, the processor 111 can be external to (i.e. separate to or remote from) the device 100. For example, in some embodiments, the processor 111 can be a separate entity or part of another device. However, it will be understood that, in other embodiments, the device 100 itself may comprise the processor 111. The processor 111 is configured to acquire the image of the skin 102 from the imaging sensor 108 and process the acquired image to measure skin pores. The processor 111 may be configured to measure skin pores using any technique suitable for measuring features in images and a person skilled in the art will be aware of such techniques. However, examples of techniques that the processor 111 can be configured to implement to measure skin pores include, but are not limited to, color contrast techniques, image segmentation techniques (such as thresholding, edge detection, or any other image segmentation technique), or any other technique suitable to measure features in images.

For example, in some embodiments, the processor 111 can be configured to measure a size of skin pores. In some of these embodiments, the processor 111 may be further configured to classify skin pores as "visible" or "enlarged". A "visible" skin pore can be any skin pore that is visible to a human eye. An "enlarged" skin pore can be any skin pore that is larger than a mean pore size for a subject. For example, a mean pore size for a female subject may be 0.3 millimeters and a mean pore size for a male subject may be 0.34 millimeters. In some embodiments, a skin pore may be classified as "visible" when the size of the skin pore is in a range from 0.35 to 0.9 millimeters. Similarly, a skin pore maybe classified as "enlarged" when the size of the skin pore is in a range from 0.62 to 0.9 millimeters. In some embodiments, the processor 11 may be configured to measure the number of skin pores (e.g. provide a pore count).

The processor 111 of the system 10 can be implemented in numerous ways, with software and/or hardware, to perform the various functions described herein. In particular implementations, the processor 111 can comprise a plurality of software and/or hardware modules, each configured to perform, or that are for performing, individual or multiple steps of the method described herein. The processor 111 may comprise one or more processors (such as one or more microprocessors, one or more multi-core processors and/or one or more digital signal processors (DSPs)), one or more processing units, and/or one or more controllers (such as one or more microcontrollers) that may be configured or programmed (e.g. using software or computer program code) to perform the various functions described herein. The processor 111 may be implemented as a combination of dedicated hardware (e.g. amplifiers, pre-amplifiers, analog-to-digital convertors (ADCs) and/or digital-to-analog convertors (DACs)) to perform some functions and a processor (e.g. one or more programmed microprocessors, controllers, DSPs and associated circuitry) to perform other functions.

As mentioned earlier, the light source 104 of the device 100 is configured to emit light 106 to illuminate skin 102 with an angle of incidence (AOI) in a range from 40 to 80 degrees. In some embodiments, for example, the light source 104 of the device 100 can be configured to emit light 106 with an angle of incidence in a range from 45 to 75 degrees, for example in a range from 50 to 70 degrees, for example in a range from 55 to 65 degrees. For example, in some embodiments, the light source 104 can be configured to emit light 106 with an angle of incidence selected from 50 degrees, 51 degrees, 52 degrees, 53 degrees, 54 degrees, 55 degrees, 56 degrees, 57 degrees, 58 degrees, 59 degrees, 60 degrees, 61 degrees, 62 degrees, 63 degrees, 64 degrees, 65 degrees, 66 degrees, 67 degrees, 68 degrees, 69 degrees and 70 degrees, or any non-integer angle of incidence between the mentioned values, or any other angle of incidence that is in a range from 40 to 80 degrees. Although some examples have been provided for the angle of incidence range and the angle of incidence for the light source 104 of the device 100, it will be understood that the light source 104 may be configured to emit light 106 with any angle of incidence in the range from 40 to 80 degrees.

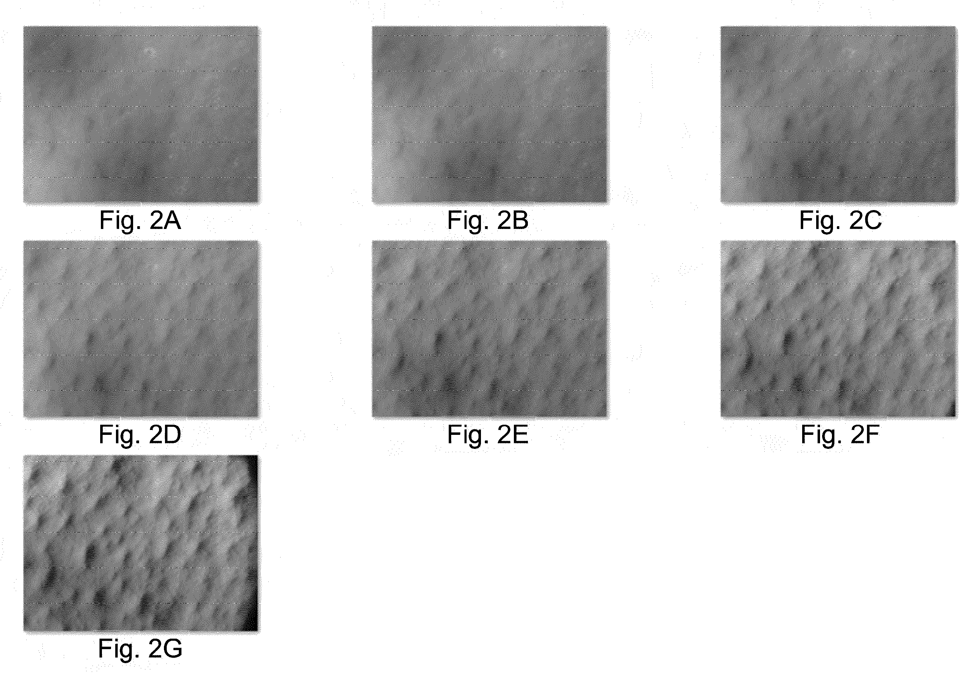

With the light source 104 of the device 100 configured to emit light 106 to illuminate skin 102 with the above-described angle of incidence, the image of the skin 102 that is acquired from the imaging sensor 108 of the device 100 has an improved resolution. In particular, the image of the skin 102 that is acquired from the imaging sensor 108 of the device 100 has a resolution that is optimal for measuring skin pores. In this way, skin pores can be better visualized and thus more accurately measured. This is illustrated in Figs. 2A-2G, which are example images acquired by an imaging sensor 108 of a device 100 with a light source 104 of the device 100 configured to emit light with different angles of incidence according to an embodiment.

Fig. 2A is an example of an image acquired by the imaging sensor 108 of the device 100 when the light source 104 of the device 100 is configured to emit light 106 with an angle of incidence of 15 degrees. Fig. 2B is an example of an image acquired by the imaging sensor 108 of the device 100 when the light source 104 of the device 100 is configured to emit light 106 with an angle of incidence of 20 degrees. Fig. 2C is an example of an image acquired by the imaging sensor 108 of the device 100 when the light source 104 of the device 100 is configured to emit light 106 with an angle of incidence of 30 degrees. Fig. 2D is an example of an image acquired by the imaging sensor 108 of the device 100 when the light source 104 of the device 100 is configured to emit light 106 with an angle of incidence of 40 degrees. Fig. 2E is an example of an image acquired by the imaging sensor 108 of the device 100 when the light source 104 of the device 100 is configured to emit light 106 with an angle of incidence of 50 degrees. Fig. 2F is an example of an image acquired by an imaging sensor of a device when a light source of the device is configured to emit light with an angle of incidence of 60 degrees. Fig. 2G is an example of an image acquired by an imaging sensor of a device when a light source of the device is configured to emit light with an angle of incidence of 70 degrees.

As illustrated in Figs. 2A-G, the images acquired by the imaging sensor 108 of the device 100 when the light source 104 of the device 100 is configured to emit light 106 with an angle of incidence in the range from 40 to 80 degrees (as illustrated in Figs. 2D-G) have a higher resolution than the image acquired by an imaging sensor of a device when a light source of the device is configured to emit light outside this range (as illustrated in Figs. 2A-2C). In particular, it can be seen from Figs. 2A-G that images acquired when the light source 104 of the device 100 is configured to emit light 106 with an angle of incidence in the range from 40 to 80 degrees have the highest pore contrast. Thus, as illustrated in Figs. 2A-G, an angle of incidence in the range from 40 to 80 degrees is optimal for resolving skin pores.

The pore contrast can be defined by the following equation:

$$\text{Pore contrast} = \left(\frac{\sigma}{mean}\right) * 100\% \,,$$

where "$\sigma$" is the standard deviation of the intensity distribution in the image and "mean" is the mean of the intensity distribution in the image. The highest pore contrast can be obtained when the standard deviation in the histogram of intensities is high due to the shadowing effects occurring in the skin pores when illuminated at different spatial locations around the imaging sensor 108. It is observed that an angle of incidence in the range from 40 to 80 degrees is optimal for measuring skin pores.

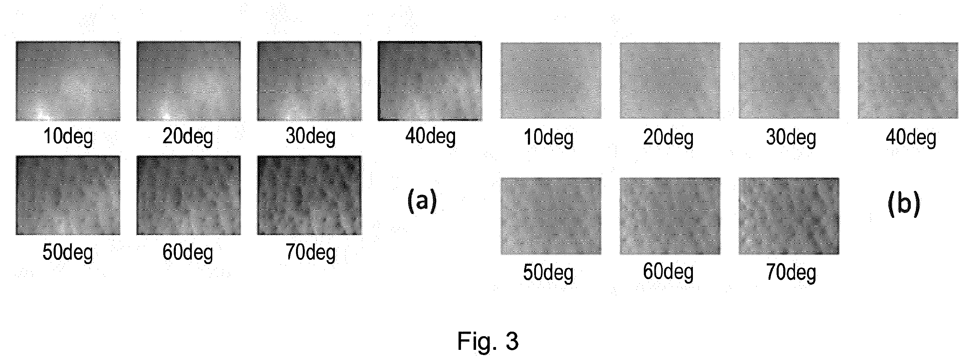

As mentioned earlier, the device 100 of the system 10 can comprise any type of the light source 104 and, in some embodiments, the light source 104 may comprise a light emitting diode (LED). In some of these embodiments, the light emitting diode may be a 3 millimeter light emitting diode, i.e. a 3 mm LED. In this way, the pore contrast in the images acquired by the imaging sensor 108 of skin 102 illuminated with such a light source 104 at an angle of incidence in a range from 40 to 80 degrees can be further improved. This is illustrated in Fig. 3. In particular, Fig. 3 illustrates example images acquired by an imaging sensor 108 of a device 100 with a light source 104 of the device 100 configured to emit light 106 with different angles of incidence according to another embodiment.

Fig. 3 (a) illustrates images acquired by an imaging sensor 108 of a device 100 with a light source 104 of the device 100 configured to emit light 106 with different angles of incidence, where the light source 104 of the device 100 comprises a 3 mm LED. Fig. 3(b) illustrates images acquired by an imaging sensor of a device with a light source of the device configured to emit light with different angles of incidence, where the light source of the device comprises a large divergence or large radiation angle LED having intensity distributed over a wide range of angles (which will be referred to as "lumiled"). As illustrated in Fig. 3, as explained earlier, the highest pore contrast is achieved in images acquired when the light source 104 of the device 100 is configured to emit light 106 with an angle of incidence in the range from 40 to 80 degrees. Moreover, it can be seen that an improved pore contrast can be achieved with a 3 mm LED (as illustrated in Fig. 3(a)) compared to the previously described lumiled (as illustrated in Fig. 3(b)).

The improved pore contrast that can be achieved using a 3 mm LED is also shown in the following table, where the pore contrast ("stdev/mean") measured for a device 100 comprising a 3 mm LED and for another device comprising the previously described lumiled is shown for different angle of incidence ("AOI"):

| AOI [deg] | mean | stdev | 3 mm led stdev/mean | lumiled stdev/mean |
|---|---|---|---|---|
| 10 | 126 | 18.0 | 14 | 4 |
| 20 | 126 | 20.1 | 16 | 5 |
| 30 | 126 | 21.2 | 17 | 6 |
| 40 | 125 | 23.0 | 18 | 7 |
| 50 | 122 | 24.2 | 20 | 9 |
| 60 | 124 | 28.3 | 23 | 12 |
| 70 | 118 | 35.4 | 30 | 19 |

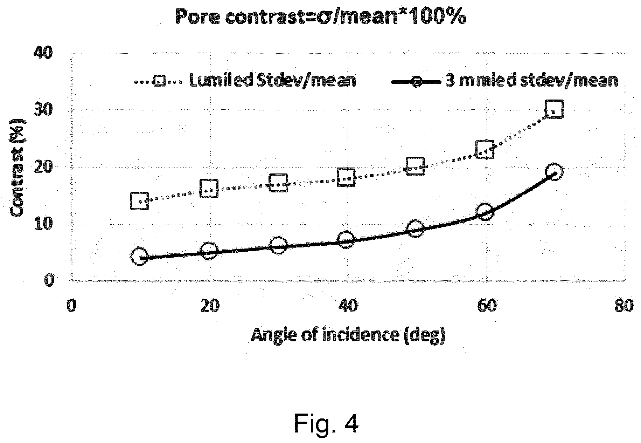

The improved pore contrast that can be achieved using a 3 mm LED is also shown in Fig. 4, which is a graphical illustration of pore contrast measured for different angles of incidence according to an embodiment. In particular, Fig. 4 illustrates the measured pore contrast ("stdev/mean") plotted against the angle of incidence ("AOI").

In some embodiments, the light source 104 of the device 100 may have a radiation angle in a particular range. The radiation angle referred to herein is the angle at which an intensity (e.g. a luminous intensity) of the light 106 emitted by the light source 104 is half that (i.e. 50%) of a maximum (e.g. peak) intensity of the light 106 emitted by the light source 104. A radiation angle is, for example, a measure of a dispersion (or radiation pattern) of light 106 emitted by a light source 104. A radiation pattern of a light source 104 viewed on-axis in a narrower viewing angle has a higher intensity than a radiation pattern of a light source 104 viewed on-axis in a wider viewing angle.

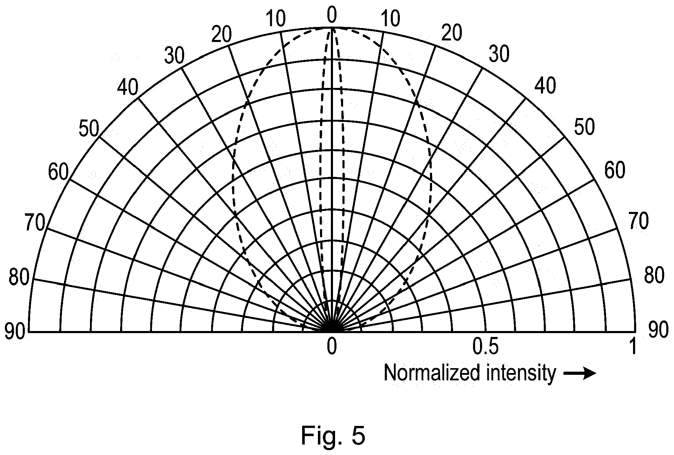

Fig. 5 illustrates a radiation angle range for the light source 104 of the device 100 according to an embodiment. As illustrated in Fig. 5, in some embodiments, the light source 104 may have a radiation angle in a range from 5 to 40 degrees. In some embodiments, for example, the light source 104 can have a radiation angle in a range from 10 to 35 degrees, for example in a range from 15 to 30 degrees, for example in a range from 20 to 25 degrees. In some embodiments, for example, the light source 104 of the device 100 can have a radiation angle in a range from 10 to 30 degrees, for example in a range from 10 to 25 degrees. For example, in some embodiments, the light source 104 of the device 100 can have a radiation angle selected from 10 degrees, 11 degrees, 12 degrees, 13 degrees, 14 degrees, 15 degrees, 16 degrees, 17 degrees, 18 degrees, 19 degrees, 20 degrees, 21 degrees, 22 degrees, 23 degrees, 24 degrees, and 25 degrees, or any non-integer radiation angle between the mentioned values, or any other radiation angle that is in a range from 5 to 40 degrees. Although some examples have been provided for the radiation angle range and the radiation angle for the light source 104 of the device 100, it will be understood that the light source 104 of the device 100 may have any radiation angle in the range from 5 to 40 degrees according to some embodiments.

In some embodiments, the light source 104 of the device 100 maybe configured to emit light 106 to uniformly (or homogeneously) illuminate the skin 102. For example, in some embodiments, the light source 104 may be configured to emit light 106 to provide a uniform (or homogenous) light intensity distribution toward the skin 102. More specifically, for example, the light source 104 may be configured to emit light 106 to uniformly illuminate the skin 102 in the field of view of the imaging sensor 108 or to provide a uniform intensity distribution of light toward the skin 102 in the field of view of the imaging sensor 108.

In some embodiments, the light source 104 of the device 100 may comprise a plurality of light emitters suitably arranged to provide uniform illumination to the skin 102. For example, the plurality of light emitters maybe provided in a circular (e.g. ring) or tiled arrangement to provide uniform illumination to the skin 102. Alternatively or in addition, the plurality of light emitters may be tilted to provide uniform illumination to the skin 102. While it has been described that the light source 104 may be configured to emit light 106 to uniformly illuminate the skin 102, it will be appreciated that in some embodiments, the light source 104 may be configured to emit light 106 to substantially uniformly illuminate the skin 102 or to emit light 106 to (e.g. substantially) uniformly illuminate an area of skin that is of a predefined size. Although some examples have been provided for the manner in which the light source 104 may be configured to emit light 106 to uniformly illuminate the skin 102, a person skilled in the art will also be aware of other examples. Thus, according to some embodiments, the illumination conditions can prevent non-linear effects or non-uniformities for intensity being present in the field of view.

Although not illustrated in Fig. 1, in some embodiments, the device 100 may further comprise an optical component positioned such that the light 106 emitted from the light source 104 passes through the optical component before illuminating the skin 102. The optical component can be configured to collimate the light 106 emitted from the light source 104 of the device 100. In some embodiments, the optical component may comprise a light guide, an off-axis lens, a periscope, a white cap with diffuse internal reflection, a Fresnel lens (such as a 25 millimeter Fresnel lens), or any other optical component, or any combination of optical components, configured to collimate the light 106 emitted from the light source 104. The optical component can be configured to guide the light 106 emitted from the light source 104 of the device 100. The light provided by the optical component results in sharper shadowing effects in the skin pores. As such, the pore contrast is further increased and thus provides improved conditions of measuring skin pores.

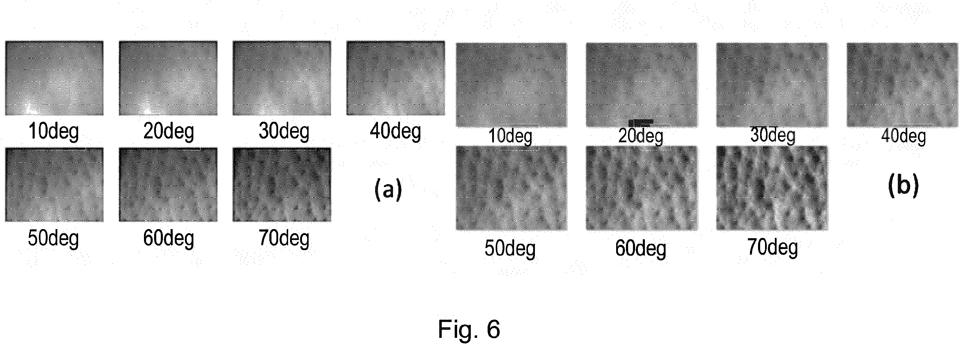

In some embodiments, the imaging sensor 108 of the device 100 may be further configured to calibrate the image of the skin 102. Alternatively, in some embodiments, the processor 111 may be configured to calibrate the image of the skin 102. For example, according to some embodiments, the image of the skin 102 may be calibrated using flat field correction. In embodiments where the image of the skin 102 is calibrated, the processor 111 can be configured to process the calibrated image to measure skin pores. The skin pores can be measured more accurately in this way, since the calibration (e.g. using flat field correction) increases pore contrast and thus provides improved conditions for the measurement. This is illustrated in Fig. 6. In particular, Fig. 6(a) illustrates example images acquired by an imaging sensor 108 of a device 100 with a light source 104 of the device 100 configured to emit light 106 with different angles of incidence without flat field correction having been applied to the images. On the other hand, Fig. 6(b) illustrates example images acquired by the imaging sensor 108 of the device 100 with the light source 104 of the device 100 configured to emit light 106 with different angles of incidence where the images have been calibrated using flat field correction.

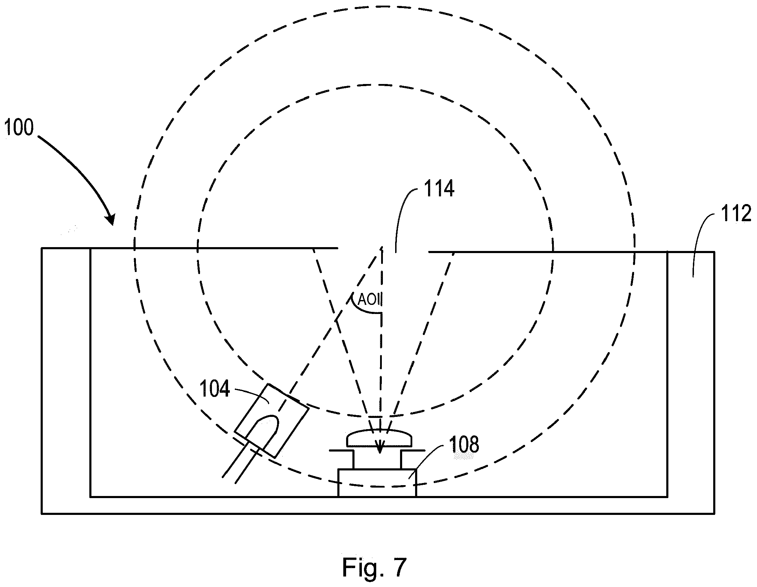

Fig. 7 illustrates the device 100 of the system 10 according to an example embodiment. The device 100 according to this illustrated example embodiment is as described earlier with reference to Fig. 1. In particular, the device 100 comprises a light source 104 configured to emit light 106 to illuminate the skin 102 and an imaging sensor 108 configured to detect light 110 reflected from the skin 102 and convert the detected light 110 into an image of the skin 102. As described earlier, the light source 104 of the device 100 is configured to emit light 106 with an angle of incidence in a range from 40 to 80 degrees. In the example embodiment illustrated in Fig. 7, the device 100 further comprises a housing 112 configured to house the light source 104 and the imaging sensor 108. The housing 112 comprises an aperture 114 through which the light source 104 is configured to emit light 106 and the imaging sensor 108 is configured to detect light 110 reflected from the skin 102.

In some embodiments, the aperture 114 can have a stop size in a range from 0.2 to 0.6 millimeters (mm). In some embodiments, for example, the aperture 114 may have a stop size in a range from 0.225 to 0.575 mm, for example in a range from 0.25 to 0.55 mm, for example in a range from 0.275 to 0.525 mm, for example in a range from 0.3 to 0.5 mm, for example in a range from 0.325 to 0.475 mm, for example in a range from 0.35 to 0.45 mm, for example in a range from 0.375 to 0.425 mm. For example, in some embodiments, the aperture 114 may have a stop size selected from 0.2 mm, 0.3 mm, 0.4 mm, 0.5 mm, 0.6 mm, or any non-integer stop size between the mentioned values, or any other stop size that is in a range from 0.2 to 0.6 mm. Although some examples have been provided for the range of stop size, it will be understood that the aperture 114 of the housing 112 of the device 100 may have any stop size in the range from 0.2 to 0.6 mm according to some embodiments.

In some embodiments, the light source 104 of the device 100 described herein may be configured to emit unpolarized light. In some of these embodiments, the device 100 may further comprise a polarizer (not illustrated). The polarizer can be configured to polarize the light 110 reflected from the skin 102. Thus, the polarizer may be positioned such that light 110 reflected from the skin 102 reaches the polarizer before the imaging sensor 108. For example, the polarizer can be positioned before (e.g. in front of) the imaging sensor 108. In embodiments where the device 100 comprises a polarizer, the imaging sensor 108 can be configured to detect the polarized light 110 reflected from the skin 102. Alternatively, in other embodiments, the light source 104 described herein may be configured to emit polarized light. In these embodiments, the imaging sensor 108 can be configured to detect the polarized light 110 reflected from the skin 102.

In some embodiments, the light source 104 of the device 100 described herein may comprise a single light emitter. In other embodiments, the light source 104 described herein may comprise a plurality of light emitters. For example, according to some embodiments, the light source 104 described herein may comprise at least two light emitters, at least three light emitters, at least four light emitters, at least five light emitters, at least six light emitters, or any other number of light emitters. In embodiments where the light source 104 comprises a plurality of light emitters, the plurality of light emitters may be configured together as a single lighting module.

In some embodiments, the plurality of light emitters may be positioned in a circular (e.g. a ring) arrangement. The light emitters configured to emit light 106 to illuminate skin 102 with an angle of incidence in a range from 40 to 80 degrees can each be angled (e.g. bent) towards the optical axis of the imaging sensor 108 in order to emit light 106 to illuminate skin 102 with this angle of incidence. In some embodiments, the light emitters can be positioned symmetrically around the imaging sensor 108. In some embodiments, for example, the light emitters may be generally evenly spaced with respect to each other around the imaging sensor 108. In some embodiments, the light source 104 may be positioned with respect to the field of view (FOV) of the imaging sensor 108, such that specular reflected light from the skin 102 is not on the imaging sensor 108. For example, the light source 104 may be positioned outside the field of view (FOV) of the imaging sensor 104.

In some embodiments comprising a plurality of light emitters, the plurality of light emitters can be configured to sequentially emit light 106 to illuminate the skin 102. For example, in some embodiments, individual ones of the plurality of light emitters may be operated (e.g. turned on) to illuminate the skin 102 sequentially (e.g. in series or consecutively). In this way, a higher pore contrast can be achieved than if the plurality of light emitters are operated to illuminate the skin 102 simultaneously. As described earlier, in some embodiments, the plurality of light emitters may be positioned in a circular arrangement around the imaging sensor 108 and can each be angled (e.g. bent) towards the optical axis of the imaging sensor 108 in order to emit light 106 to illuminate skin 102 with an angle of incidence in a range from 40 to 80 degrees. In these embodiments, where the plurality of light emitters are configured to sequentially emit light 106 to illuminate the skin 102, the pore contrast will be higher since the skin pores will be illuminated from different sides at different times.

In some embodiments, the light source 104 (and thus the one or more light emitters) may be provided (or mounted) on a substrate, such as a semiconductor substrate or a printed circuit board (PCB). Thus, according to some embodiments, the light source 104 may be a chip-on-board (COB) light source. In some embodiments where the light source 104 comprises a plurality of light emitters, the plurality of light emitters may be provided (or mounted) on the same substrate. The plurality of light emitters may comprise any type of light emitter or any combination of types of light emitter, such as any of the types provided earlier in respect of the light source 104 of the device 100. For example, the plurality of light emitters may comprise any one or more of a semiconductor light emitter (such as a light emitting diode LED, a resonant cavity light emitting diode RCLED, a vertical cavity laser diode VCSEL, an edge emitting laser, or any other semiconductor light emitter, or any combination of semiconductor light emitter), an organic light emitter (such as a passive-matrix PMOLED, an active-matrix AMOLED, or any other organic light emitter, or any combination of organic light emitters), a solid state light emitter, or any other light emitter, or any combination of light emitters.

In some embodiments, the light source 104 of the device 100 described herein may be configured to emit visible light, such as white light. In some embodiments where the light source 104 is configured to emit visible light, the visible light may have a wavelength in a range from 389 nm to 780 nm. In some embodiments where the light source 104 is configured to emit white light, the white light may have a correlated color temperature (CCT) in a range from 2000 K to 20000 K, for example in a range from 2700 K to 20000 K, for example in a range from 2700 K to 6500 K. Alternatively or in addition to visible light, in some embodiments, the light source 104 described herein may be configured to emit infrared (IR) light, such as near infrared (NIR) light. In some embodiments where the light source 104 is configured to emit infrared light, the infrared light may have a wavelength in a range from 750 nm to 3000 nm. Although some examples have been provided for the type of light source 104, it will be appreciated that the device 100 may alternatively or in addition comprise any other type of light source or light sources suitable to illuminate the skin 102.

In some embodiments, a distance between the light source 104 and the imaging sensor 108 of the device 100 described herein may be greater than or equal to 12 millimeters (mm). In some embodiments, a distance between the light source 104 and the imaging sensor 108 of the device 100 described herein may be in a range from 12 to 30 mm, for example in a range from 13 to 29 mm, for example in a range from 14 mm to 28 mm, for example in a range from 15 mm to 27 mm, for example in a range from 16 mm to 26 mm, for example in a range from 17 to 25 mm, for example in a range from 18 mm to 24 mm, for example in a range from 19 mm to 23 mm, for example in a range from 20 mm to 22 mm. For example, in some embodiments, the distance between the light source 104 and the imaging sensor 108 maybe a distance selected from 12 mm, 13 mm, 14 mm, 15 mm, 16 mm, 17 mm, 18 mm, 19 mm, 20 mm, 21 mm, 22 mm, 23 mm, 24 mm, 25 mm, 26 mm, 27 mm, 28 mm, 29 mm, 30 mm, or any non-integer distance between the mentioned values, or any other distance greater than or equal to 12 mm. In any of embodiments described herein, the imaging sensor 108 of the device 100 described herein may have a particular depth of focus (DOF). For example, in some embodiments, the imaging sensor 108 may have a depth of focus (DOF) that is greater than or equal to 12 mm.

Although the device 100 has been described herein with reference to Figs. 1 and 7, it will be appreciated that Figs. 1 and 7 only show the components required to illustrate certain embodiments and, in a practical implementation, the device 100 may comprise other components or additional components to those shown.

For example, although not illustrated in Figs. 1 and 7, the device 100 may comprise a communications interface (or communications circuitry). The communications interface can be for enabling the device 100 or components of the device 100 to communicate with and/or connect to one or more other devices, components, interfaces, memories, or sensors (such as any of those described herein). For example, the communications interface can be for enabling the imaging sensor 108 of the device 100 to communicate with and/or connect to the processor described earlier for image processing. The communications interface may be configured to communicate wirelessly, via a wired connection, or via any other communication (or data transfer) mechanism. In some wireless embodiments, the communications interface may, for example, use radio frequency (RF), Bluetooth, or any other wireless communication technologies, for communications.

Although also not illustrated in Figs. 1 and 7, the device 100 may comprise a memory, or may be configured to communicate with and/or connect to a memory external to (i.e. separate to or remote from) the device 100. The memory may comprise any type of non-transitory machine-readable medium, such as cache or system memory including volatile and non-volatile computer memory such as random access memory (RAM), static RAM (SRAM), dynamic RAM (DRAM), read-only memory (ROM), programmable ROM (PROM), erasable PROM (EPROM), and electrically erasable PROM (EEPROM). The memory can be configured to store program code that can be executed by a processor to cause the device 100 to operate in the manner described herein. Alternatively or in addition, the memory can be configured to store information acquired by the device 100. For example, in some embodiments, the memory may be configured to store an image of skin acquired by the imaging sensor 108 of the device 100, a skin pore measurement obtained by the processor 111, or any other information, or any combination of information resulting from the method described herein.

Although also not illustrated in Figs. 1 and 7, the device 100 may comprise a user interface, or may be configured to communicate with and/or connect to a user interface external to (i.e. separate to or remote from) the device 100. The user interface can be configured to render (or output, display, or provide) information resulting from the method described herein. For example, in some embodiments, the user interface may be configured to render (or output, display, or provide) an image of skin acquired by the imaging sensor 108, a skin pore measurement obtained by the processor 111, or any other information, or any combination of information resulting from the method described herein. Alternatively or in addition, the user interface can be configured to receive a user input. For example, the user interface may allow a user to manually enter information or instructions, interact with and/or control the device 100. Thus, the user interface may be any user interface that enables the rendering (or outputting, displaying, or providing) of information and, alternatively or in addition, enables a user to provide a user input.

For example, the user interface may comprise one or more switches, one or more buttons, a keypad, a keyboard, a mouse, a touch screen or an application (for example, on a smart device such as a tablet, a smartphone, or any other smart device), a display or display screen, a graphical user interface (GUI) or any other visual component, one or more speakers, one or more microphones or any other audio component, one or more lights (such as light emitting diode LED lights), a component for providing tactile or haptic feedback (such as a vibration function, or any other tactile feedback component), an augmented reality device (such as augmented reality glasses, or any other augmented reality device), a smart device (such as a smart mirror, a tablet, a smart phone, a smart watch, or any other smart device), or any other user interface, or combination of user interfaces. In some embodiments, the user interface that is controlled to render information may be the same user interface as that which enables the user to provide a user input.

Although also not illustrated in Figs. 1 and 7, the device 100 may comprise a battery or other power supply for powering the device 100 or means for connecting the device 100 to a mains power supply, or any other component, or any combination of components.

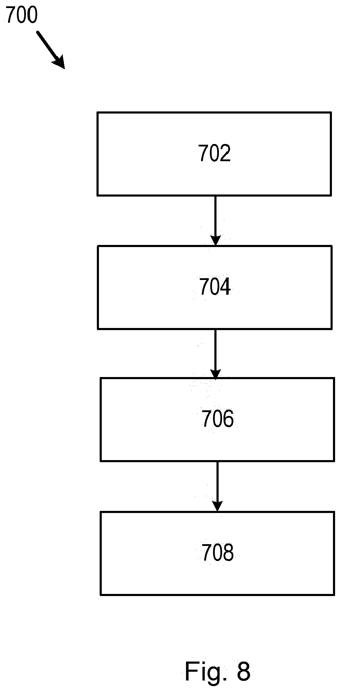

Fig. 8 illustrates a method 700 for measuring skin pores according to an embodiment. The method 700 may generally be performed by or under the control of the processor 111 of the system 10. At block 702, light 106 is emitted from the light source 104 of the device 100 to illuminate the skin 102 with an angle of incidence in a range from 40 to 80 degrees. At block 704, light 110 reflected from the skin 102 is detected by the imaging sensor 108 of the device 100. At block 706, the detected light 110 is converted by the imaging sensor 108 into an image of the skin. At block 708, the image of the skin 102 is processed to measure skin pores, as described earlier.

There is also provided a computer program product comprising a computer readable medium. The computer readable medium has computer readable code embodied therein. The computer readable code is configured such that, on execution by a suitable computer or processor, the computer or processor is caused to perform the method described herein. The computer readable medium may be, for example, any entity or device capable of carrying the computer program product. For example, the computer readable medium may include a data storage, such as a ROM (such as a CD-ROM or a semiconductor ROM) or a magnetic recording medium (such as a hard disk). Furthermore, the computer readable medium may be a transmissible carrier, such as an electric or optical signal, which may be conveyed via electric or optical cable or by radio or other means. When the computer program product is embodied in such a signal, the computer readable medium may be constituted by such a cable or other device or means. Alternatively, the computer readable medium may be an integrated circuit in which the computer program product is embedded, the integrated circuit being adapted to perform, or used in the performance of, the method described herein.

There is thus provided herein an improved system and method for imaging skin and, more specifically, an improved system, method and computer program product for measuring skin pores. According to these aspects, images of improved resolution (and thus improved quality) can be acquired. The resolution of the images achieved by the system 10 described herein is optimum for adequately resolving skin pores. This is possible through use of one or more light sources 104 having a large angle of incidence and optionally also the use of a small radiation angle light source 104 (e.g. with maximum intensity distribution on the optical axis) and/or the one or more light sources 104 being configured to provide a uniform light intensity distribution (e.g. in a narrow range of angles in the field of view). Thus, according to the aspects and embodiments described herein, images acquired by the imaging sensor 108 of the device 100 can be used by the processor 111 of the system 10 to improve the accuracy of skin pore measurements. In this way, it is possible to provide more accurate skin monitoring and to more reliably detect skin conditions.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1. A system (10) for measuring skin pores, the system (10) comprising:
   a device (100), wherein the device (100) comprises:

   a light source (104) configured to emit light (106) to illuminate skin (102) with an angle of incidence in a range from 40 to 80 degrees; and
   an imaging sensor (108) configured to detect light (110) reflected from the skin (102) and convert the detected light (110) into an image of the skin (102),

   wherein the system further comprises:
   a processor (111) configured to acquire the image of the skin (102) from the imaging sensor (108) and process the acquired image to measure skin pores.

2. A system (10) as claimed in claim 1, wherein the light source (104) is configured to emit light (106) to illuminate the skin (102) with an angle of incidence in a range from 50 to 70 degrees.

3. A system (10) as claimed in any one of claims 1 or 2, wherein the light source (104) has a radiation angle in a range from 5 to 40 degrees, wherein the radiation angle is the angle at which an intensity of the light emitted by the light source (104) is half that of a maximum intensity of the light (106) emitted by the light source (104).

4. A system (10) as claimed in claim 3, wherein the light source (104) has a radiation angle in a range from 10 to 30 degrees.

5. A system (10) as claimed in any one of the preceding claims, wherein the light source (104) is configured to emit light (106) to uniformly illuminate the skin.

6. A system (10) as claimed in any one of the preceding claims, wherein the imaging sensor (108) is further configured to calibrate the image of the skin using flat field correction.

7. A system (10) as claimed in any one of the preceding claims, wherein the light source (104) comprises a plurality of light emitters.

8. A system (10) as claimed in claim 7, wherein the plurality of light emitters are positioned in a circular arrangement.

9. A system (10) as claimed in any one of claims 7 or 8, wherein the plurality of light emitters are configured to sequentially emit light (106) to illuminate the skin (102).

10. A system (10) as claimed in any one of the preceding claims, wherein the device (100) further comprises:
an optical component positioned such that the light (106) emitted from the light source (104) passes through the optical component before illuminating the skin (102), wherein the optical component is configured to collimate the light (106) emitted from the light source (104).

11. A system (10) as claimed in claim 10, wherein the optical component comprises any one or more of: a light guide, an off-axis lens, a periscope, a white cap with diffuse internal reflection, and a Fresnel lens.

12. A system (10) as claimed in any one of the preceding claims, wherein the device (100) further comprises:

a housing (112) configured to house the light source (104) and the imaging sensor (108),
wherein the housing (112) comprises an aperture (114) through which the light source (104) is configured to emit light (106) and the imaging sensor (108) is configured to detect light (110) reflected from the skin (102), wherein the aperture (114) has a stop size in a range from 0.2 millimeters to 0.6 millimeters.

13. A system (10) as claimed in any one of the preceding claims, wherein a distance between the light source (104) and the imaging sensor (106) is greater than 12 millimeters.

14. A method for measuring skin pores, the method comprising:

emitting light (106) to illuminate skin (102) with an angle of incidence in a range from 40 to 80 degrees;
detecting light (110) reflected from the skin (102);
converting the detected light (110) into an image of the skin (102); and
processing the image of the skin (102) to measure skin pores.

15. A computer program product comprising a computer readable medium, the computer readable medium having computer readable code embodied therein, the computer readable code being configured such that, on execution by a suitable computer or processor, the computer or processor is caused to perform the method as claimed in claim 14.

10

100

| 104 | 108 | 111 |

106          110

102

Fig. 1

Fig. 2A  Fig. 2B  Fig. 2C

Fig. 2D  Fig. 2E  Fig. 2F

Fig. 2G

Fig. 3

Fig. 4

Fig. 5

EP 3 536 224 A1

10deg 20deg 30deg 40deg

50deg 60deg 70deg

**(a)**

10deg 20deg 30deg 40deg

50deg 60deg 70deg

**(b)**

Fig. 6

Fig. 7

EP 3 536 224 A1

700

```
      ┌─────────────────────┐
      │                     │
      │        702          │
      │                     │
      └──────────┬──────────┘
                 │
                 ▼
      ┌─────────────────────┐
      │                     │
      │        704          │
      │                     │
      └──────────┬──────────┘
                 │
                 ▼
      ┌─────────────────────┐
      │                     │
      │        706          │
      │                     │
      └──────────┬──────────┘
                 │
                 ▼
      ┌─────────────────────┐
      │                     │
      │        708          │
      │                     │
      └─────────────────────┘
```

Fig. 8

Europäisches
Patentamt

European
Patent Office

Office européen
des brevets

# EUROPEAN SEARCH REPORT

Application Number

EP 18 16 0467

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2016/262624 A1 (NAKAJIMA HIROE [JP] ET AL) 15 September 2016 (2016-09-15) * paragraph [0014] - paragraph [0073] * * paragraph [0107] - paragraph [0114] * * figure 2 * | 1-15 | INV. A61B5/00 |
| Y | US 2006/182323 A1 (KOLLIAS NIKIFOROS [US] ET AL) 17 August 2006 (2006-08-17) * the whole document * | 1-15 | |
| A | US 2013/256505 A1 (GOMI SHINICHIRO [JP] ET AL) 3 October 2013 (2013-10-03) * paragraph [0090] - paragraph [0091] * * figure 9 * | 1-15 | |
| A | US 2003/026110 A1 (SATOH MASAO [JP] ET AL) 6 February 2003 (2003-02-06) * the whole document * | 1-15 | |

TECHNICAL FIELDS
SEARCHED      (IPC)

A61B

The present search report has been drawn up for all claims

| Place of search | Date of completion of the search | Examiner |
|---|---|---|
| Berlin | 27 August 2018 | Abraham, Volkhard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 16 0467

This annex lists the patent family members relating to the patent documents cited in the above-mentioned European search report.
The members are as contained in the European Patent Office EDP file on
The European Patent Office is in no way liable for these particulars which are merely given for the purpose of information.

27-08-2018

| Patent document cited in search report | | Publication date | Patent family member(s) | | Publication date |
|---|---|---|---|---|---|
| US 2016262624 | A1 | 15-09-2016 | CN | 105592792 A | 18-05-2016 |
| | | | JP | 5731724 B1 | 10-06-2015 |
| | | | JP | 6295229 B2 | 14-03-2018 |
| | | | JP | 2015144902 A | 13-08-2015 |
| | | | JP | 2018122104 A | 09-08-2018 |
| | | | JP | WO2015060376 A1 | 09-03-2017 |
| | | | TW | 201531992 A | 16-08-2015 |
| | | | US | 2016262624 A1 | 15-09-2016 |
| | | | WO | 2015060376 A1 | 30-04-2015 |
| US 2006182323 | A1 | 17-08-2006 | BR | PI0600902 A | 03-10-2006 |
| | | | CA | 2536855 A1 | 17-08-2006 |
| | | | EP | 1693003 A1 | 23-08-2006 |
| | | | US | 2006182323 A1 | 17-08-2006 |
| US 2013256505 | A1 | 03-10-2013 | CN | 103356167 A | 23-10-2013 |
| | | | JP | 6051558 B2 | 27-12-2016 |
| | | | JP | 2013205571 A | 07-10-2013 |
| | | | US | 2013256505 A1 | 03-10-2013 |
| US 2003026110 | A1 | 06-02-2003 | JP | 3626433 B2 | 09-03-2005 |
| | | | JP | 2003050422 A | 21-02-2003 |
| | | | US | 2003026110 A1 | 06-02-2003 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8346347 B